# EUROPEAN PATENT APPLICATION

(11) **EP 2 348 029 A1**
(43) Date of publication of application: **27.07.2011**
(21) Application number: 11159814.0
(22) Date of filing: 21.07.2005
(51) Int. Cl.: C07H 19/073, C07H 19/173

(54) **Preparation of alkyl-substituted 2-deoxy-2-fluoro-d-ribofuranosyl pyrimidines and purines and their derivatives**

(30) Priority: 21.07.2004 US 589866 P; 09.09.2004 US 608320 P
(62) Divisional of application: 05775359.2
(71) Applicant: Pharmasset, Inc., Tucker, GA 30084 (US)
(72) Inventor: Wang, Peiyuan, Glenrock, NJ 07452 (US); Chun, Byoung-Kwon, Robbinsville, NJ New Jersey 08691 (US); Shi, Junxing, Duluth, GA Georgia 30096 (US); Du, Jinfa, New Hope, PA Pennsylvania 18938 (US)
(74) Representative: Portal, Frédéric

(57) **Abstract**

The present invention provides a method to prepare anti-HCV nucleosides containing the 2-deoxy-2-fluoro-2-C-methyl-β-D-ribofuranosyl nucleosides from a preformed, preferably naturally-occurring, nucleoside.

## Description

This application is being filed on 21 July 2005 as a PCT International Patent application in the name of Pharmasset, Inc., a U.S. national corporation, applicant for the designation of all countries except the US, and Pieyang Wang, a citizen of China, Wojciech Stec, a citizen of Poland, Jeremy Clark, a citizen of the US, Byoung-Kown Chun, a citizen of Republic of Korea, Junxing Shi, a citizen of China, and Jinfa Du, a citizen of the US, all applicants for the designation of the US only, and claims priority to U.S. Provisional Patent Application No. 60/589,866, filed July 21, 2004.

### FIELD OF THE INVENTION

The present invention provides (i) a process for preparing a 2-deoxy-2-fluoro-2-methyl-D-ribonolactone derivative, (ii) conversion of the lactone to nucleosides with potent anti- HCV activity, and their analogues, and (iii) a method to prepare the anti-HCV nucleosides containing the 2'-deoxy-2'-fluoro-2'-C-methyl-β-D-ribofuranosyl nucleosides from a preformed, preferably naturally-occurring, nucleoside.

### BACKGROUND OF THE INVENTION

In light of the fact that HCV infection has reached epidemic levels worldwide, and has tragic effects on the infected patients. Presently there is no universally effective treatment for this infection and the only drugs available for treatment of chronic hepatitis C are various forms of alpha interferon (IFN-α), either alone or in combination with ribavirin. However, the therapeutic value of these treatments has been compromised largely due to adverse effects, which highlights the need for development of additional options for treatment.

HCV is a small, enveloped virus in the *Flaviviridae* family, with a positive single-stranded RNA genome of -9.6 kb within the nucleocapsid. The genome contains a single open reading frame (ORF) encoding a polyprotein of just over 3,000 amino acids, which is cleaved to generate the mature structural and nonstructural viral proteins. ORF is flanked by 5' and 3' non-translated regions (NTRs) of a few hundred nucleotides in length, which are important for RNA translation and replication. The translated polyprotein contains the structural core (C) and envelope proteins (El, E2, p7) at the N-terminus, followed by the nonstructural proteins (NS2, NS3, NS4A, NS4B, NS5A, NS5B). The mature structural proteins are generated via cleavage by the host signal peptidase. The junction between NS2 and NS3 is autocatalytically cleaved by the NS2/NS3 protease, while the remaining four junctions are cleaved by the N-terminal serine protease domain of NS3 complexed with NS4A. The NS3 protein also contains the NTP-dependent helicase activity which unwinds duplex RNA during replication. The NS5B protein possesses *RNA-dependent RNA polymerase* (RDRP) activity, which is essential for viral replication. It is emphasized here that, unlike HBV or HIV, no DNA is involved in the replication of HCV.

U. S. Patent Application (Serial No. 10/828,753) discloses that 1-(2-deoxy-2-fluoro-2-C-methyl-β-D-ribofuranosyl)cytosine (**14**) is a potent and selective anti-HCV agent. The original synthetic procedures (Schemes 1-3) are quite inefficient, with overall yields at or below 4% and are not amenable to large-scale.

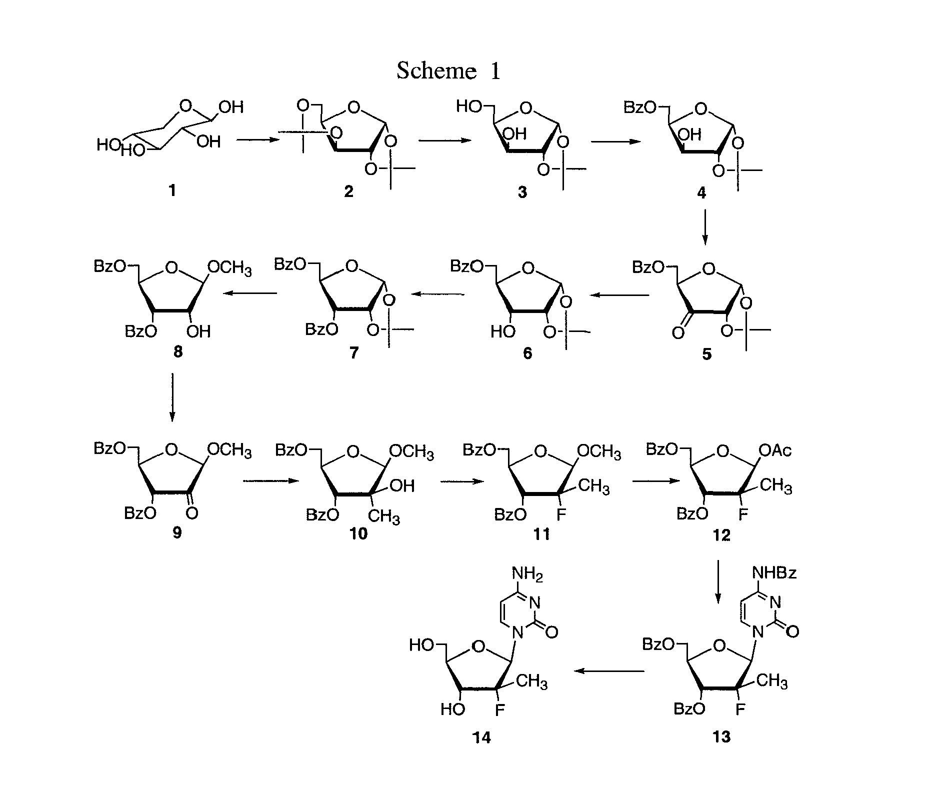

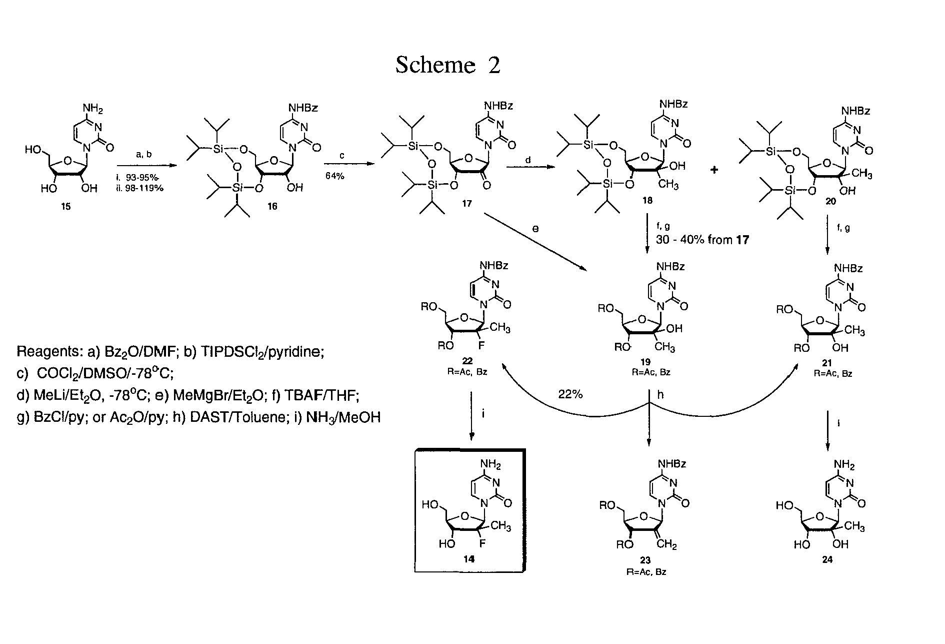

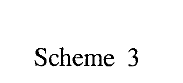

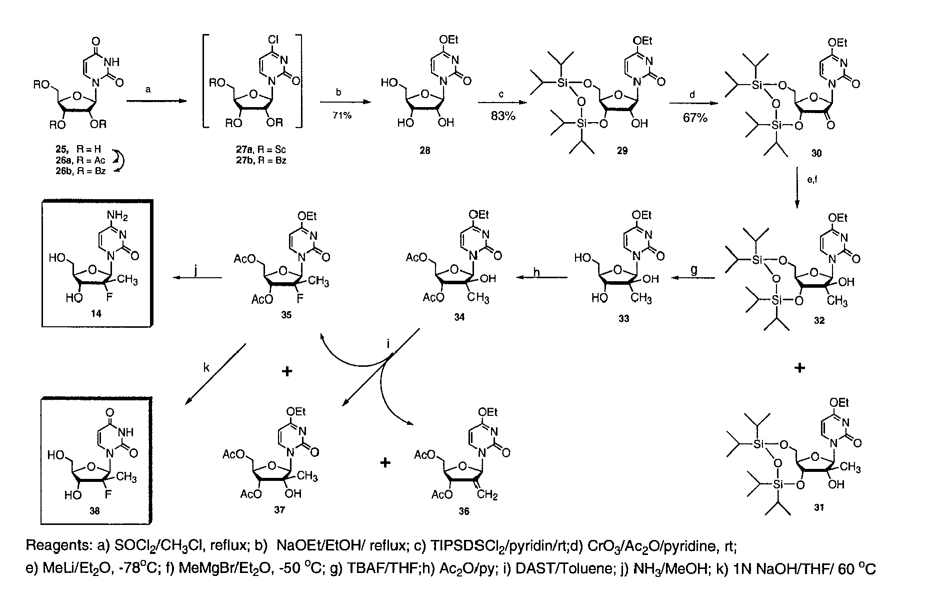

What is needed is a novel and cost effective process for the synthesis of 2-*C-*alkyl-2-deoxy-2-substituted-D-ribopyranosyl nucleosides that have activity against HCV.

### SUMMARY OF INVENTION

The present invention as disclosed herein relates to the composition and synthetic methods of compounds of general formulas [I] and [II], 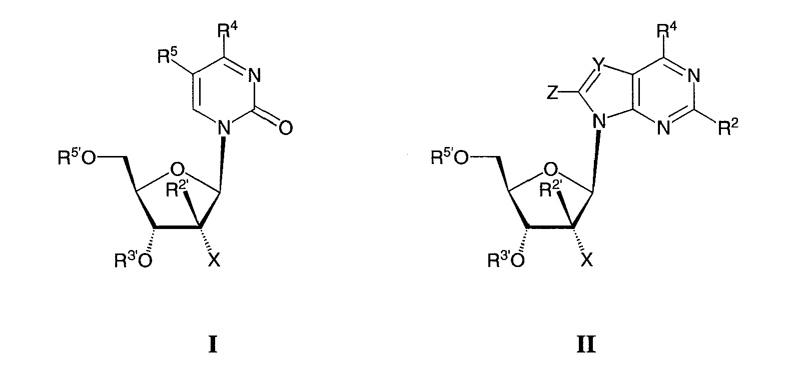 wherein
X is halogen (F, Cl, Br),
Y is N or CH,
Z is, halogen, OH, OR', SH, SR', NH₂, NHR', or R'
R^{2'} is alkyl of C₁-C₃, vinyl, or ethynyl;
R^{3'} and R^{5'} can be same or different H, alkyl, aralkyl, acyl, cyclic acetal such as 2',3'-O-isopropylidene or 2',3-O-benzylidene, or 2',3'-cyclic carbonate;
R², R⁴, R⁵ and R⁶ are independently H, halogen including F, Cl, Br, I, OH,
OR', SH, SR', N₃, NH₂, NHR', NR'₂, NHC(O)OR', lower alkyl of C₁-C₆, halogenated (F, Cl, Br, I) lower alkyl of C₁-C₆ such as CF₃ and CH₂CH₂F, lower alkenyl of C₂-C₆ such as CH=CH₂, halogenated (F, Cl, Br, I) lower alkenyl of C₂-C₆ such as CH=CHCl, CH=CHBr and CH=CHI, lower alkynyl of C₂-C₆ such as C≡CH, halogenated (F, Cl, Br, I) lower alkynyl of C₂-C₆, hydroxy lower alkyl of C₁-C₆ such as CH₂OH and CH₂CH₂OH, halogenated (F, Cl, Br, I) lower alkyl of C₁-C₆, lower alkoxy of C₁-C₆ such as methoxy and ethoxy, CO₂H,
CO₂R', CONH₂, CONHR', CONR'₂, CH=CHCO₂H, CH=CHCO₂R'; and,
R' is an optionally substituted alkyl of C₁-C₁₂ (particularly when the alkyl is an amino acid residue), cycloalkyl, optionally substituted alkynyl of C₂-C₆, optionally substituted lower alkenyl of C₂-C₆, or optionally substituted acyl.

In other aspects, the present invention provides methods to prepare nucleosides containing the 2-deoxy-2-fluoro-2-*C*-methyl-D-ribofuranosyl moiety of general structures of **III** and **IV,** 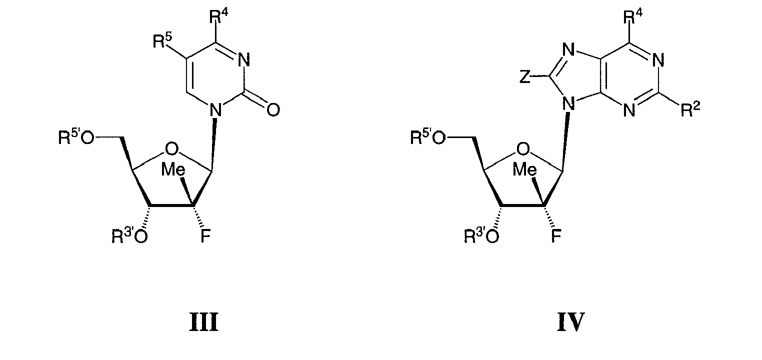 through (i) synthesis of the 3,5-protected 2-deoxy-2-fluoro-2-*C*-methyl-D-ribono-γ-lactone intermediate of general structure **V**, (ii) conversion of **V** into purine and pyrimidine nucleosides of general structures of **III** and **IV,** and (iii) preparation of nucleosides of general structures of **III** and **IV** from preformed, preferably natural, nucleosides.

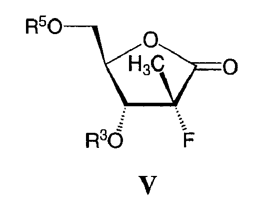

Regarding **III, IV** and **V** above, R⁴ and R⁵ are as defined above and R³ and R⁵ can be independently H, Me, Acyl (such as Ac, Bz, substituted Bz), benzyl, substituted benzyl, Trityl, Trialkylsilyl, *t*-Butyldialkylsilyl, *t*-Butyldiphenylsilyl, TIPDS, THP, MOM, MEM, or R³ and R⁵ are linked through **-SiR₂-O-SiR₂-** or -SiR₂-, wherein R is a lower alkyl group such as Me, Et, *n*-Pr or *i*-Pr.

Still another aspect of the present invention are the novel lactone intermediates of formula V and processes for the preparation of the lactone intermediates as detailed below, including precursor ester intermediates as also detailed below.

### DETAILED DESCRIPTION

Presently no preventive means against Flaviviridae, including hepatitis C virus (HCV), Dengue virus (DENV), West Nile virus (WNV) or Yellow Fever virus (YFV), infection is available. The only approved therapies are for treatment of HCV infection with alpha interferon alone or in combination with the nucleoside ribavirin, but the therapeutic value of these treatments has been compromised largely due to adverse effects. It was recently discovered that a group of nucleosides, including 2'-deoxy-2'-fluoro-2'-C-methylcytidine (**14**), exhibit potent and selective activity against replication of HCV in a replicon system. However, the difficulty of chemical synthesis of this and analogous nucleosides impedes further biophysical, biochemical, pharmacological evaluations mandatory for development of clinical drugs for treatment of Flaviviridae infection.

The present invention provides an efficient preparation of nucleosides containing the 2-deoxy-2-fluoro-2-*C*-methyl-D-ribofuranosyl moiety **III** and **IV,** through (i) synthesis of intermediate the 3,5-protected 2-deoxy-2-fluoro-2-*C*-methyl-D-ribono-γ-latone of general structure **V**, (ii) conversion of **V** into purine and pyrimidine nucleosides of general structures of **III** and **IV**, and (iii) preparation of nucleosides of general structures of **III** and **IV** from preformed, preferably natural,
nucleosides.

### Definitions

The term "independently" is used herein to indicate that the variable, which is independently applied, varies independently from application to application. Thus, in a compound such as R^{a}XYR^{a}, wherein R^{a} is "independently carbon or nitrogen", both R^{a} can be carbon, both R^{a} can be nitrogen, or one R^{a} can be carbon and the other R^{a} nitrogen.

As used herein, the terms "enantiomerically pure" or "enantiomerically enriched"refers to a nucleoside composition that comprises at least approximately 95%, and preferably approximately 97%, 98%, 99% or 100% of a single enantiomer of that nucleoside.

As used herein, the term "substantially free of" or "substantially in the absence of" refers to a nucleoside composition that includes at least 85 or 90% by weight, preferably 95% to 98% by weight, and even more preferably 99% to 100% by weight, of the designated enantiomer of that nucleoside. In a preferred embodiment, in the methods and compounds of this invention, the compounds are substantially free of enantiomers.

The term "alkyl," as used herein, unless otherwise specified, refers to a saturated straight or branched hydrocarbon chain of typically C₁ to C₁₀, and specifically includes methyl, ethyl, propyl, isopropyl, butyl, isobutyl, *t*-butyl, pentyl, , isopentyl, neopentyl, hexyl, isohexyl, cyclohexyl, cyclohexylmethyl, 3-methylpentyl, 2,2-dimethylbutyl, and 2,3-dimethylbutyl, and the like. The term includes both substituted and unsubstituted alkyl groups. Alkyl groups can be optionally substituted with one or more moieties selected from the group consisting of hydroxyl, amino, alkylamino, arylamino, alkoxy, aryloxy, nitro, cyano, sulfonic acid, sulfate, phosphonic acid, phosphate, or phosphonate. One or more of the hydrogen atoms attached to carbon atom on alkyl may be replaces by one or more halogen atoms, e.g. fluorine or chlorine or both, such as trifluoromethyl, difluoromethyl, fluorochloromethyl, and the like. The hydrocarbon chain may also be interrupted by a heteroatom, such as N, O or S.

The term "lower alkyl," as used herein, and unless otherwise specified, refers to a C₁ to C₄ saturated straight or branched alkyl group, including both substituted and unsubstituted forms as defined above. Unless otherwise specifically stated in this application, when alkyl is a suitable moiety, lower alkyl is preferred. Similarly, when alkyl or lower alkyl is a suitable moiety, unsubstituted alkyl or lower alkyl is preferred.

The term "cycloalkyl", as used herein, unless otherwise specified, refers to a saturated hydrocarbon ring having 3-8 carbon atoms, preferably, 3-6 carbon atoms, such as cyclopropyl, cyclobutyl, cyclopentyl and cyclohexyl. The cycloalkyl group may also be substituted on the ring by and alkyl group, such as cyclopropylmethyl and the like.

The terms "alkylamino" or "arylamino" refer to an amino group that has one or two alkyl or aryl substituents, respectively.

The term "protected," as used herein and unless otherwise defined, refers to a group that is added to an oxygen, nitrogen, or phosphorus atom to prevent its further reaction or for other purposes. A wide variety of oxygen and nitrogen protecting groups are known to those skilled in the art of organic synthesis. Non-limiting examples include: C(O)-alkyl, C(O)Ph, C(O)aryl, CH₃, CH₂-alkyl, CH₂-alkenyl, CH₂Ph, CH₂-aryl, CH₂O-alkyl CH₂O-aryl, SO₂-alkyl SO₂-aryl *tert*butyldimethylsilyl, *tert*-butyldiphenylsilyl, and 1,3-(1,1,3,3-tetraisopropyldisiloxanylidene).

The term "aryl," as used herein, and unless otherwise specified, refers to phenyl, biphenyl, or naphthyl, and preferably phenyl. The term includes both substituted and unsubstituted moieties. The aryl group can be substituted with one or more substituents, including, but not limited to hydroxyl, halo, amino, alkylamino, arylamino, alkoxy, aryloxy, nitro, cyano, sulfonic acid, sulfate, phosphonic acid, phosphate, or phosphonate, either unprotected, or protected as necessary, as known to those skilled in the art, for example, as taught in T.W. Greene and P.G.M. Wuts, "Protective Groups in Organic Synthesis," 3rd ed., John Wiley & Sons, 1999.

The terms "alkaryl" or "alkylaryl" refer to an alkyl group with an aryl substituent. The terms "aralkyl" or "arylalkyl" refer to an aryl group with an alkyl substituent, as for example, benzyl.

The term "halo," as used herein, includes chloro, bromo, iodo and fluoro.

The term "acyl ester" or "O-linked ester" refers to a carboxylic acid ester of the formula C(O)R' in which the non-carbonyl moiety of the ester group, R', is a straight or branched alkyl, or cycloalkyl or lower alkyl, alkoxyalkyl including methoxymethyl, aralkyl including benzyl, aryloxyalkyl such as phenoxymethyl, aryl including phenyl optionally substituted with halogen (F, Cl, Br, I), C₁ to C₄ alkyl or C₁ to C₄ alkoxy, sulfonate esters such as alkyl or aralkyl sulphonyl including methanesulfonyl, the mono, di or triphosphate ester, trityl or monomethoxytrityl, substituted benzyl, trialkylsilyl (e.g. dimethyl-t-butylsilyl) or diphenylmethylsilyl. Aryl groups in the esters optimally include a phenyl group.

The term "acyl" refers to a group of the formula R"C(O)-, wherein R" is a straight or branched alkyl, or cycloalkyl, amino acid, aryl including phenyl, alkylaryl, aralkyl including benzyl, alkoxyalkyl including methoxymethyl, aryloxyalkyl such as phenoxymethyl; or substituted alkyl (including lower alkyl), aryl including phenyl optionally substituted with chloro, bromo, fluoro, iodo, C₁ to C₄ alkyl or C₁ to C₄ alkoxy, sulfonate esters such as alkyl or aralkyl sulphonyl including methanesulfonyl, the mono, di or triphosphate ester, trityl or monomethoxy-trityl, substituted benzyl, alkaryl, aralkyl including benzyl, alkoxyalkyl including methoxymethyl, aryloxyalkyl such as phenoxymethyl. Aryl groups in the esters optimally comprise a phenyl group. In particular, acyl groups include acetyl, trifluoroacetyl, methylacetyl, cyclopropylacetyl, cyclopropyl carboxy, propionyl, butyryl, hexanoyl, heptanoyl, octanoyl, neo-heptanoyl, phenylacetyl, 2-acetoxy-2-phenylacetyl, diphenylacetyl, α-methoxy-α-trifluoromethyl-phenylacetyl, bromoacetyl, 2-nitro-benzeneacetyl, 4-chloro-benzeneacetyl, 2-chloro-2,2-diphenylacetyl, 2-chloro-2-phenylacetyl, trimethylacetyl, chlorodifluoroacetyl, perfluoroacetyl, fluoroacetyl, bromodifluoroacetyl, methoxyacetyl, 2-thiopheneacetyl, chlorosulfonylacetyl, 3-methoxyphenylacetyl, phenoxyacetyl, tert-butylacetyl, trichloroacetyl, monochloro-acetyl, dichloroacetyl, 7H-dodecafluoro-heptanoyl, perfluoro-heptanoyl, 7H-dodecafluoroheptanoyl, 7-chlorododecafluoro-heptanoyl, 7-chloro-dodecafluoro-heptanoyl, 7H-dodecafluoroheptanoyl, 7*H*-dodeca-fluoroheptanoyl, nona-fluoro-3,6-dioxaheptanoyl, nonafluoro-3,6-dioxaheptanoyl, perfluoroheptanoyl, methoxybenzoyl, methyl 3-amino-5-phenylthiophene-2-carboxyl, 3,6-dichloro-2-methoxy-benzoyl, 4-(1,1,2,2-tetrafluoro-ethoxy)-benzoyl, 2-bromo-propionyl, omega-aminocapryl, decanoyl, n-pentadecanoyl, stearyl, 3-cyclopentyl-propionyl, 1 -benzene-carboxyl, O-acetylmandelyl, pivaloyl acetyl, 1-adamantane-carboxyl, cyclohexane-carboxyl, 2,6- pyridinedicarboxyl, cyclopropane-carboxyl, cyclobutane-carboxyl, perfluorocyclohexyl carboxyl, 4-methylbenzoyl, chloromethyl isoxazolyl carbonyl, perfluorocyclohexyl carboxyl, crotonyl, 1-methyl-1H-indazole-3-carbonyl, 2-propenyl, isovaleryl, 1-pyrrolidinecarbonyl, 4-phenylbenzoyl. When the term acyl is used, it is meant to be a specific and independent disclosure of acetyl, trifluoroacetyl, methylacetyl, cyclopropylacetyl, propionyl, butyryl, hexanoyl, heptanoyl, octanoyl, neo-heptanoyl, phenylacetyl, diphenylacetyl, cttrifluoromethyl-phenylacetyl, bromoacetyl, 4-chloro-benzeneacetyl, 2-chloro-2,2-diphenylacetyl, 2-chloro-2-phenylacetyl, trimethylacetyl, chlorodifluoroacetyl, perfluoroacetyl, fluoroacetyl, bromodifluoroacetyl, 2-thiopheneacetyl, tert-butylacetyl, trichloroacetyl, monochloro-acetyl, dichloroacetyl, methoxybenzoyl, 2-bromo-propionyl, decanoyl, n-pentadecanoyl, stearyl, 3-cyclopentyl-propionyl, 1 - benzene-carboxyl, pivaloyl acetyl, 1-adamantane-carboxyl, cyclohexane-carboxyl, 2,6-pyridinedicarboxyl, cyclopropane-carboxyl, cyclobutane-carboxyl, 4-methylbenzoyl, crotonyl, 1-methyl-1H-indazole-3-carbonyl, 2-propenyl, isovaleryl, 4-phenylbenzoyl.

The term "lower acyl" refers to an acyl group in which R", above defined, is lower alkyl.

The term "purine" or "pyrimidine" base includes, but is not limited to, adenine, N⁶-alkylpurines, N⁶-acylpurines (wherein acyl is C(O)(alkyl, aryl, alkylaryl, or arylalkyl), N⁶-benzylpurine, N⁶-halopurine, N⁶-vinylpurine, N⁶-acetylenic purine, N⁶-acyl purine, N⁶-hydroxyalkyl purine, N⁶-allcylaminopurine, N6-thioallcyl purine, N²-alkylpurines, N2-alkyl-6-thiopurines, thymine, cytosine, 5-fluorocytosine, 5-methylcytosine, 6-azapyrimidine, ncluding 6-azacytosine, 2-and/or 4-mercaptopyrmidine, uracil, 5-halouracil, including 5-fluorouracil, C⁵-alkylpyrimidines, C -benzylpyrimidines, C⁵-halopyrimidines, C⁵-vinylpyrimidine, C⁵-acetylenic pyrimidine, C⁵-acyl pyrimidine, C⁵-hydroxyalkyl purine, C⁵-amidopyrimidine, C⁵-cyanopyrimidine, ,C⁵-iodopyrimidine, C⁶-Iodo-pyrimidine, C⁵-Br-vinyl pyrimidine, C⁶-Br-vinyl pyriniidine, C⁵-nitropyrimidine, C⁵-amino-pyrimidine, N²-alkylpurines, N²-alkyl-6-thiopurines, 5-azacytidinyl, 5-azauracilyl, triazolopyridinyl, imidazolopyridinyl, pyrrolopyrimidinyl, and pyrazolopyrimidinyl. Purine bases include, but are not limited to, guanine, adenine, hypoxanthine, 2,6-diaminopurine, and 6-chloropurine. Functional oxygen and nitrogen groups on the base can be protected as necessary or desired. Suitable protecting groups are well known to those skilled in the art, and include trimethylsilyl, dimethylhexylsilyl, *t-*butyldimethylsilyl, and *t*-butyldiphenylsilyl, trityl, alkyl groups, and acyl groups such as acetyl and propionyl, methanesulfonyl, and p-toluenesulfonyl.

The term "amino acid" includes naturally occurring and synthetic α, β γ or δ amino acids, and includes but is not limited to, amino acids found in proteins, i.e. glycine, alanine, valine, leucine, isoleucine, methionine, phenylalanine, tryptophan, proline, serine, threonine, cysteine, tyrosine, asparagine, glutamine, aspartate, glutamate, lysine, arginine and histidine. In a preferred embodiment, the amino acid is in the L-configuration. Alternatively, the amino acid can be a derivative of alanyl, valinyl, leucinyl, isoleucinyl, prolinyl, phenylalaninyl, tryptophanyl, methioninyl, glycinyl, serinyl, threoninyl, cysteinyl, tyrosinyl, asparaginyl, glutaminyl, aspartoyl, glutaroyl, lysinyl, argininyl, histidinyl, β-alanyl, β-valinyl, β-leucinyl, β-isoleucinyl, β-prolinyl, β-phenylalaninyl, β-tryptophanyl, β-methioninyl, β-glycinyl, β-serinyl, β-threoninyl, β-cysteinyl, β-tyrosinyl, β-asparaginyl, β-glutaminyl, β-aspartoyl, β-glutaroyl, β-lysinyl, β-argininyl or β-histidinyl. When the term amino acid is used, it is considered to be a specific and independent disclosure of each of the esters of α, β γ or δ glycine, alanine, valine, leucine, isoleucine, methionine, phenylalanine, tryptophan, proline, serine, threonine, cysteine, tyrosine, asparagine, glutamine, aspartate, glutamate, lysine, arginine and histidine in the D and L-configurations.

The term "pharmaceutically acceptable salt or prodrug" is used throughout the specification to describe any pharmaceutically acceptable form (such as an ester, phosphate ester, salt of an ester or a related group) of a compound which, upon administration to a patient, provides the active compound. Pharmaceutically acceptable salts include those derived from pharmaceutically acceptable inorganic or organic bases and acids. Suitable salts include those derived from alkali metals such as potassium and sodium, alkaline earth metals such as calcium and magnesium, among numerous other acids well known in the pharmaceutical art. Pharmaceutically acceptable salts may also be acid addition salts when formed with a nitrogen atom. Such salts are derived from pharmaceutically acceptable inorganic or organic acids, such as hydrochloric, sulfuric, phosphoric, acetic, citric, tartaric, and the like. Pharmaceutically acceptable prodrugs refer to a compound that is metabolized, for example hydrolyzed or oxidized, in the host to form the compound of the present invention. Typical examples of prodrugs include compounds that have biologically labile protecting groups on a functional moiety of the active compound. Prodrugs include compounds that can be oxidized, reduced, aminated, deaminated, hydroxylated, dehydroxylated, hydrolyzed, dehydrolyzed, alkylated, dealkylated, acylated, deacylated, phosphorylated, dephosphorylated to produce the active compound.

### PREPARATION OF THE COMPOUNDS

### (i) Synthesis of 3,5-Di-O-protected-D-ribono-γ-lactone

Wittig reaction of 2,3-*O*-isopropylidene-D-glyceraldehyde **39** (Scheme 4) with commercially available **40** affords the (*E*)-product **41** as a major product. Sharpless dihydroxylation (J. Org. Chem. 1992, 57, 2768-2771) using AD-mix-β as a dihydroxylation reagent gives only the desired product **42** in very high yield. High yield lactonization of **42** to 2-*C*-methyl-D-arabino-γ-lactone (**46**) is achieved by HCl/MeOH treatment. Selective *O*-benzoylation of primary and secondary OH groups yields 3,5-di-*O*-benzol derivative **47** in high yield. Treatment of **47** with DAST or Deoxofluor, [bis(2-methoxyethyl)amino]sulfur trifluoride, under various conditions gives trace amounts of the desired 2'-fluoro-ribono-γ-lactone **49,** but mostly a mixture from which the non-fluorinated ribonolactone (**48**) is isolated. However, treatment of **47** with excess, preferably three (3) equivalents, of tertiary amine, preferably diisopropylethylamine, and excess, preferably five (5) equivalents, of DAST or Deoxofluor provides **49** in ∼50% yield. It was also found that using 3,5-O-MOM instead of benzoyl protection, the yield of **48** approaches 90%. Thus, treatment of **46** with dimethoxymethane in the presence of strong acid such as trifluoromethylsulfonic acid affords **50,** which upon reaction with DAST or Deoxofluor in the presence of base yielded 87% isolated yield of **49.**

It was also discovered that smooth fluorination can occur upon treatment of the open-chain monobenzoate **43,** which can be readily obtained by selective benzoylation of **42,** with DAST or Deoxofluor giving rise to the desired ethyl 2-deoxy-2-fluoro-2-*C*-methyl-3-*O*-benzoyl-4,5-*O*-isopropylidene-D-ribonate **44.** Lactonization of **44** gives only the γ-lactone **45.** Further benzoylation of **45** affords dibenzoate **49.**

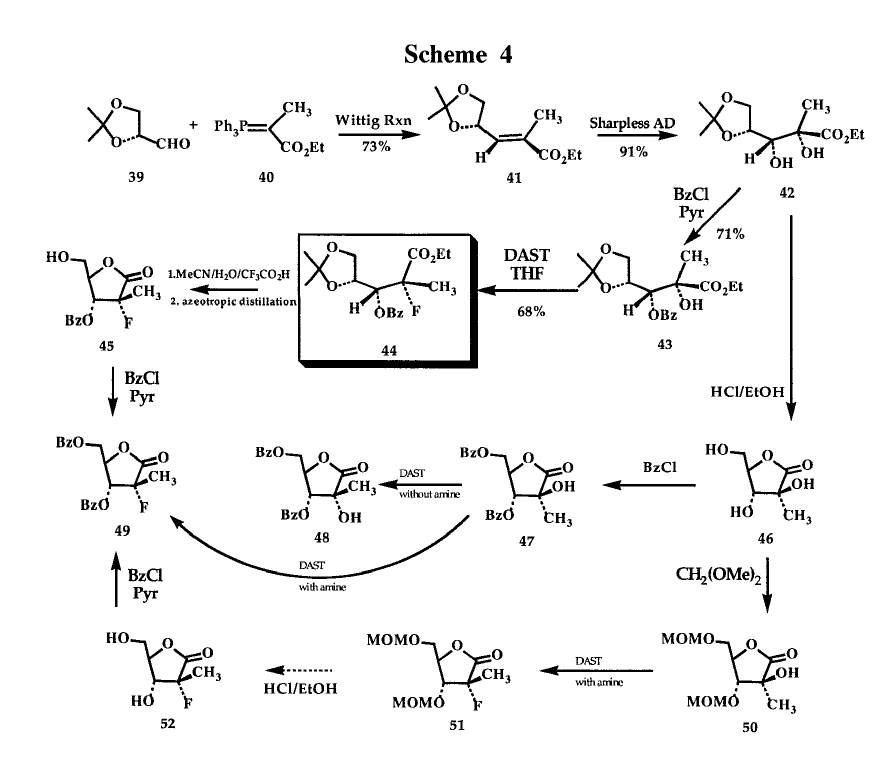

In one embodiment of the present invention, a method is provided for the synthesis of intermediate **49** through **Reformatsky** condensation of **39** with an alkyl 2-bromopropionate such as **53** (Scheme 5) in the presence of activated zinc in an ethereal solvent such as diethyl ether or tetrahydrofuran (or a mixture of the two solvents) to give **54,** which is converted to **55** by oxidation. Possible oxidizing agents are: activated dimethylsulfoxide, such as a mixture of dimethylsulfoxide, trifluoroacetic anhydride or acetic anhydride (a Swern/Moffat oxidiation); chromium trioxide or other chromate reagent; Dess-Martin periodinane; or tetrapropylammonium perruthenate (TPAP) with or without molecular sieves. This oxidation to provide the C-3 ketone preferably proceeds without affecting the stereochemistry at C-4.

Fluorination of **55** is performed at the 2-position using an electrophilic fluorination ("F⁺") in an appropriate solvent such as dimethylformamide, tetrahydrofuran, ethanol, *tert*-butanol, or diethyl ether or any combination of these solvents known to those skilled in the art (Rozen, et. al., J. Org. Chem., 2001, 66, 7646-7468; Jun-An Ma and Dominique Cahard, Journal of Fluorine Chemistry, 2004**,** *in press,* and references cited therein), to afford **56.** Some non-limiting examples of electrophilic fluorinating reagents are Selectflour^{®}, N-fluorosulfonimide (NFSI), and AcOF. Stereoselective fluorination can be achieved by using a catalyst such as an asymmetric transition metal complex catalyst as taught by Sodeoka, et al. (JP2004010555) or by other catalysts. The starting β-keto ester **55** may also be first converted to a ketene silyl acetal prior to fluorination (Rozen, et. al., J. Org. Chem., 2001, 66, 7646-7468).

Selective reduction of the C-3 ketone **56** using triphenylsilane in the presence of a Lewis acid such as AlCl₃ or in the presence of an organic acid such as trifluoroacetic acid (Kitazume, et al., J. Org. Chem., 1987, 52, 3218-3223) provides two 2,3 *anti* products **57** and **58.** However, by utilizing a stereoselective fluorination combined with the selective reduction, a good yield (with high diastereomeric excess) of **58** can be achieved. Benzoylation of **58** gives **44** which is converted to lactone **45** as described earlier.

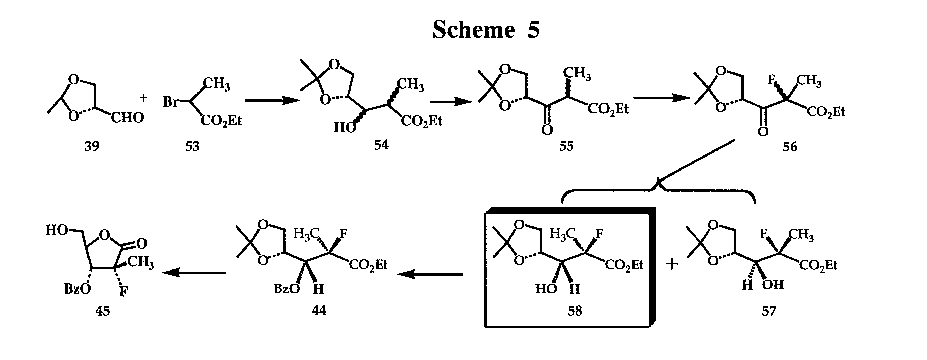

### (ii) Preparation of nucleosides containing 2-deoxy-2-fluoro-3-methyl-D-ribofuranosyl moiety by condensation.

A lactone such as **49** can be reduced to the corresponding sugar with DIBAL-H. After acetylation of the anomeric hydroxyl group, **59** (Scheme 12) is obtained in high yield. Condensation of **59** with silylated base (*e.g*., silylated *N*⁴-benzoylcytosine under Vorbrüggen's conditions) affords a mixture of protected anomeric nucleosides **60** and **60-a.** After separation of the anomers, the desired β-nucleoside **14** is prepared by deprotection with metal alcoholate in alcohol, preferably NaOMe/MeOH, or methanolic ammonia.

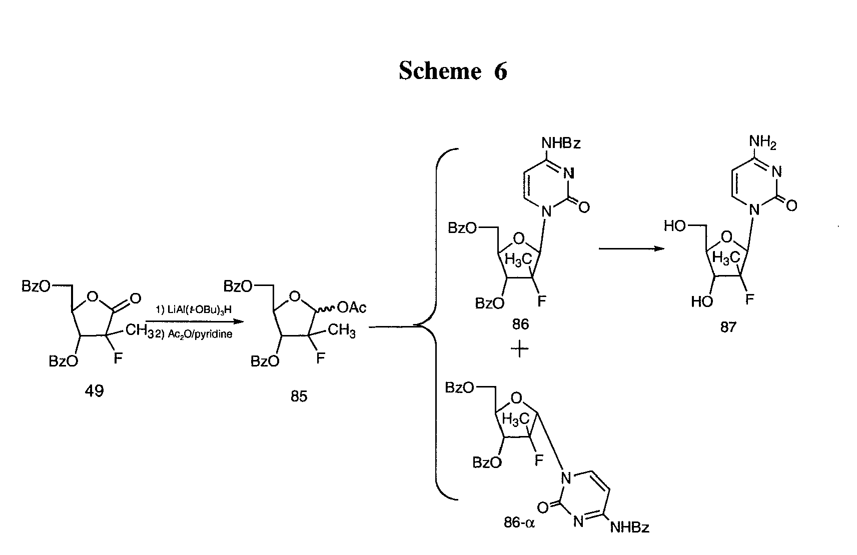

Compound **59** can be converted into the bromo sugar **61,** (Scheme 7) which is condensed with a sodium salt of purine, e.g., sodio-N6-benzoyladenine to give the corresponding protected purine nucleoside **62.** The desired free nucleoside **63** is readily obtainable by saponification.

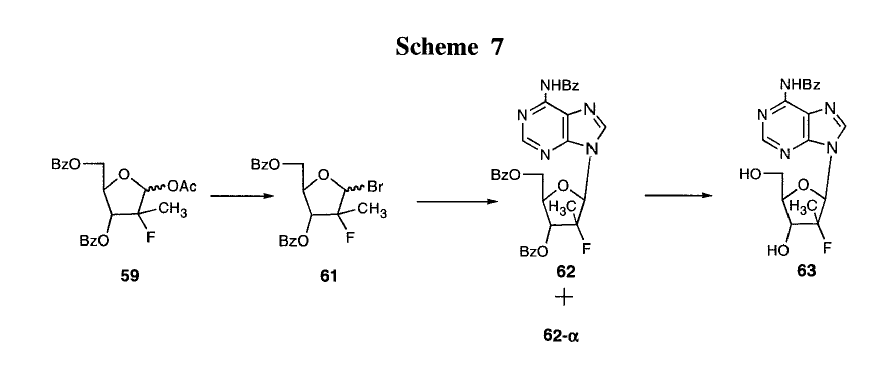

### (iii) Synthesis from preformed nucleosides:

Using preformed nucleosides as starting materials for preparation of the desired 2'-C-alkyl-2'-deoxy-2'-fluoro-β-D-ribonucleosides has certain advantages, as the formation of anomers and their subsequent separation can be circumvented, resulting in high yields of the targeted nucleosides.

Two procedures to prepare the desired nucleoside **14** from nucleoside starting materials have been disclosed (Schemes 2 and 3). As mentioned earlier, however, these procedures also produced two undesirable products **22** and **23,** the latter produced by neighboring group participation as shown in Scheme 8. The separation of the desired nucleoside **14** from the mixture is rather cumbersome. Thus, this invention prevents production of **23** using non-participating protecting group, such as THP, methyl, ethyl, benzyl, p-methoxybenzyl-, benzyloxymethyl, phenoxymethyl, methoxymethyl, ethoxymethyl, mesyl, tosyl, trifluoroacetyl, trichloroacetyl, at the 3'-OH group.

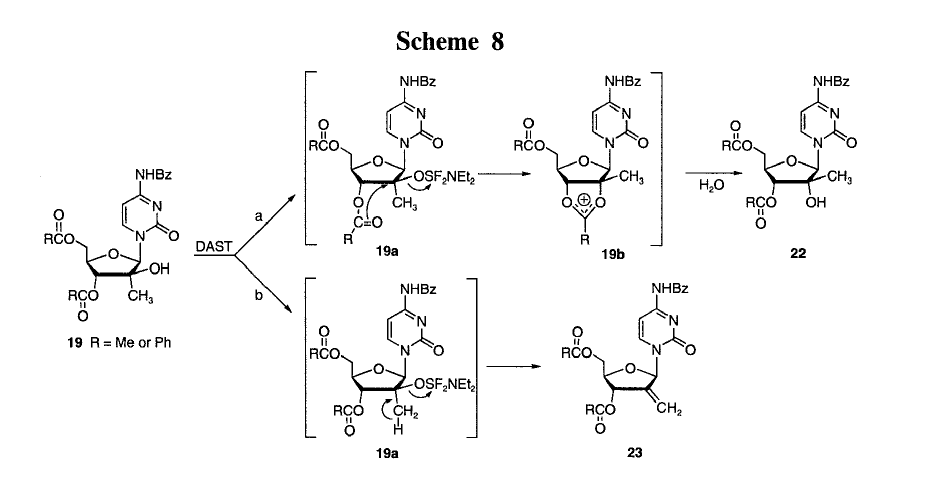

An example is shown in Scheme 17. When *N*⁴,5'-O-dibenzoyl-3'-O-mesyl-2'-deoxy-2'-*C*-methyl-β-D-arabinofuranosylcytosine (**64**) is treated with DAST or Deoxofluor, the desired fluorinated product **65** is obtained in 54% yield along **with** the olefin **66** in 39% yield. As expected, no unfluorinated cytidine derivative **67** is formed in detectable amounts. There are several ways to de-protect **65** to **14.** An example is shown in Scheme 9 that requires a double inversion of the 3'-configuration.

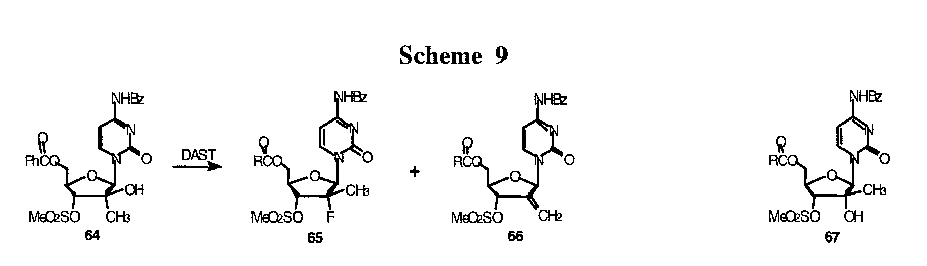

When the 3'-O-substituent is a non-participating and non-leaving group, such as methoxymethyl (MOM), methyl, benzyl, methoxybenzyl or tetrahydropyranyl, the intermediate is fluorinated more effectively than **64.**

The following examples are presented to illustrate the present invention but are not to be limited thereto.

**Experimental:** 2,3-O-Isopropylidene-D-glyceraldehyde (**39**) is prepared by literature procedures (Organic Synthesis, Annual Volume 72, page 6; J. Org. Chem. 1991, 56, 4056-4058) starting from commercially available protected D-mannitol. Other reagents, including **40** and AD-mix-β, are from commercial sources.

### EXAMPLES

### EXAMPLE 1

### Ethyl trans-2,3-dideoxy-4,5-O-isopropylidene-2-C-methyl-D-glycero-pent-2-enonate (41)

To a solution of (carbethoxyethylidene)triphenylphosphorane (**40**, 25 g, 69 mmol) in dry CH₂Cl₂ (65 mL) at room temperature is added dropwise a solution of 2,3-O-isopropylidene-D-glyceraldehyde (**39**, 9.41 g, 72.3 mmol) in CH₂Cl₂ ( 30 mL). The mixture is stirred at room temperature overnight. The reaction mixture is then concentrated to dryness, diluted with light petroleum ether (300 mL), and kept at room temperature for 2 h. Triphenylphosphine oxide precipitated is removed by filtration and an aliquot is concentrated in vacuo. The residue is purified by silica gel column chromatography with 0-1.5% EtOAc in hexanes to give **41** (10.4 g, 71%) as an oil (Carbohydrate Res., 115, 250-253 (1983)). ¹H NMR (CDCl₃) δ 1.30 (t, *J* = 6.8 Hz, 3H, -OCH₂CH₃), 1.41 (,s, 3H, CH₃), 1.45 (,s, 3H, CH₃), 1.89 (d, *J* = 1.2 Hz, 3H, 2-CH₃), 3.63 (t, *J* = 8.0 Hz, 1H, H-5), 4.14-4.23(m, 3H, H-5' and - OCH₂CH₃), 4.86 (dd, *J* = 7.6 and 13.6 Hz, 1 H, H-4), 6.69 (dd, *J* = 1.6 and 8.0 Hz, 1 H, H-3),

### EXAMPLE 2

### (2S, 3R)-3-[(4R)-2,2-Dimethyl-[1,3]dioxolan-4-yl]-2,3-dihydroxy-2-methyl-propionic acid ethyl ester (42)

A round-bottomed flask, equipped with a magnetic stirrer, is charged with 25 mL of *t-*BuOH, 25 mL of water, and 7.0 g of AD-mix-β. Stirring at room temperature produced two clear phases; the lower aqueous phase appears bright yellow. Methanesulfonamide (475 mg) is added at this point. The mixture is cooled to 0 °C whereupon some of the dissolved salts precipitated, 1.07 g (5 mmol) of **41** is added at once, and the heterogeneous slurry is stirred vigorously at 0 °C for 24 h. After this time, while the mixture is stirred at 0 °C, solid sodium sulfite (7.5 g) is added and the mixture allowed to warm to room temperature and stirred for 30-60 min. EtOAc (50 mL) is added to the reaction mixture, and after separation of the layers, the aqueous phase is further extracted with EtOAc. The organic layer is dried over Na₂SO₄ and concentrated to dryness. The residue is purified by silica gel column chromatography with 20 % EtOAc in hexanes to provide **42** (1.13 g, 91%) as a solid.
¹H NMR (DMSO-d₆) □ 1.18 (t, *J* = 6.8 Hz, 3H, -OCH₂CH₃), 1.24 (,s, 3H, CH₃), 1.25 (,s, 3H, CH₃), 1.28 (s, 3H, 2-CH3), 3.67 (t, *J* = 7.2 Hz, 1 H), 3.85, 4.06 and 4.12 (m, 4 H), 4.96 (s, 1H, 2-OH, D₂O exchangeable), 5.14 (d, *J* = 7.6 Hz, 2-OH, D₂O exchangeable). Anal. Calcd for C₁₁H₂₀O₆: C, 53.22; H, 8.12; Found: C, 53.32; H, 8.18.

### EXAMPLE 3

### (2S,3R)-3-[(4R)-2,2-Dimethyl-[1,3]dioxolan-4-yl]-3-benzoyloxy-2-hydroxy-2-methylpropionic acid ethyl ester (43)

To a solution of compound **42** (245 mg, 0.99 mmol) in dry pyridine (3 mL) is added dropwise a solution of BzCl (300 mg, 2.1 mmol) in pyridine **(1** mL). After the mixture is stirred at room temperature for 2 h, the reaction is quenched with H₂O (1 mL). The mixture is concentrated to dryness and the residue is partitioned between CH₂Cl₂ and sat. NaHCO₃ solution. The organic phase is dried (anh. Na₂SO₄), filtered and concentrated. The residue is purified by silica gel column chromatography with 5 % EtOAc in hexanes to give **43** (247 mg, 71%) as a solid. Anal. Calcd for C₁₈H2₄O₇: C, 61.35; H, 6.86; Found: C, 60.95; H, 6.73.

### EXAMPLE 4

### (2R, 3R)-3-[(4R)-2,2-Dimethyl-[1,3]dioxolan-4-yl]-3-benzoyloxy-2-fluoro-2-methyl-propionic acid ethyl ester (44)

To a solution of compound **43** (36 mg, 0.102 mmol) in anhydrous THF (1.5 mL) is added DAST or Deoxofluor (0.08 mL, 0.68 mmol) at 0 °C under argon. The reaction mixture is stirred at room temperature for 3 h, then cooled down to 0 °C, and carefully treated with cold saturated NaHCO₃ solution (2 mL). The organic layer is dried over Na₂SO₄ and concentrated to dryness. The residue is purified by silica gel column chromatography with 1-3 % EtOAc in hexanes to give **44** (24.6 mg, 68%) as a syrup. HR-FAB MS; Obsd: m/z 361.1621. Calcd for C₁₈H₂₃0₆FLi: m/z 361.1639 (M+H)⁺.

### EXAMPLE 5

### 3-O-Benzoyl-2-methyl-2-deoxy-2-fluoro-D-ribono-γ-actone (45)

A mixture of compound **44** (308 mg, 0.86 mmol), MeCN (20 mL), water (1 mL) and CF₃CO₂H (0.17 mL) is refluxed at 80-85 °C for 3 h. The open-chain intermediate is not isolated, but converted directly to **45** by azeotropic distillation using a Dean-Stark water separator. The removed MeCN is replaced with dry toluene, and the azeotropic distillation continued until the oil bath temperature reached 130 °C. Stirring at 130 °C is continued overnight. The mixture is then cooled to room temperature and the solvent is removed *in vacuo* to give a syrup, which is purified by silica gel column chromatography with 10-15 % EtOAc in hexanes to give, after solvents evaporation, solid **45** (136 mg, 58.3%).

### EXAMPLE 6

### 3,5-Di-O-benzoyl-2-methyl-2 -deoxy-2-fluoro-D-ribono-γ-lactone (49)

To a solution of **45** (60 mg, 0.224 mmol) in EtOAc (1 mL) are added pyridine (100 mg, 1.26 mmol) and 4-dimethylaminopyridine (2.7 mg). The mixture is warmed to 60 °C and BzCl (110 mg, 0.79 mmol) in EtOAc (0.4 mL) is added dropwise. After stirring for 3 h, the mixture is cooled to 0 °C and pyridine HCl salt is filtered off. The filtrate is diluted with EtOH and the mixture is evaporated to dryness. The residue is purified by silica gel column chromatography with 3-6 % EtOAc in hexanes to provide, after solvents evaporation, solid **49** (75 mg, 91 %).

### EXAMPLE 7

### 2-Methyl-D-arabino-y-lactone (46)

A solution of compound **42** (248 mg, 1 mmol) in 1.5 mL of EtOH is treated with 0.3 mL of concentrated HCl. The reaction mixture is stirred at room temperature for 2 h. The solvent is removed in vacuo (bath temp. < 45 °C). The residue is co-evaporated with toluene (3 x 10 mL) to give a residue, which is purified by silica gel column chromatography with 70 % EtOAc in hexanes. Evaporation of solvents give oily **46** (170 mg, 105%). Anal. Calcd for C₆H₁₀O₅: C, 41.24; H, 6.22; Found: C, 41.00; H, 6.74.

### EXAMPLE 8

### 3,5-Di-O-benzoyl-2-methyl-D-arabino-γ-lactone (47)

To a stirred solution of compound **46** (880 mg, 5.4 mmol) in dry pyridine (80 mL) is added dropwise a solution of BzCl (1.73 g, 12.29 mmol) in dry pyridine (45 mL) at room temperature over a period 75 min. The mixture is stirred for another 90 min, then treated with MeOH (5 mL), and concentrated to dryness. The residue is purified by silica gel column chromatography with 12-20 % EtOAc in hexanes to give **47** (1.1 g, 55%) as an oil.

### EXAMPLE 9

### 3,5-Di-O-benzoyl-2-deoxy-2-fluoro-2-C-methyl-D-ribonolactone (49)

To a solution of **47** (430 mg, 1.16 mmol) in anhydrous THF (20 mL) and diisopropylethylamine (1 mL, 5.74 mmol) is added DAST or DEOXOFLUOR (0.48 mL, 3.66 mmol) at room temperature under argon. The reaction mixture is stirred at room temperature for 3 h, then cooled down to 0 °C, and carefully treated with cold saturated NaHCO₃ solution (5 mL). The reaction mixture is partitioned between EtOAc (100 mL) and water (20 mL). The organic layer is dried over (Na₂SO₄) and concentrated to dryness. The residue is purified by silica gel column chromatography with 3-6 % EtOAc in hexanes to provide **49** (220 mg, 51 %) as a solid.

### EXAMPLE 10

### 3,5-Di-O-benzoyl-2-methyl-D-ribono-lactone (48)

To a solution of **47** (160 mg, 0.432 mmol) in anhydrous CH₂Cl₂ (5 mL) is added DAST or DEOXOFLUOR (0.15 mL, 1.14 mmol) at 0-5 °C under argon. The reaction mixture is stirred at 0-5 °C for 1 h then at room temperature. After 24 hrs, the reaction still does not go well as there is no major less polar product appears in the TLCs. The reaction mixture is cooled to 0 °C, and carefully treated with cold saturated NaHCO₃ solution. The organic layer is dried over Na₂SO₄ and concentrated to dryness. The residue is checked by proton NMR. It shows that the major product is 3,5-dibenzoyl-2-methyl-D-ribono-γ-lactone (**48**), which is identical with authentic sample. Traces of **49** are detected on the spectrum.

### EXAMPLE 11

### 3,5-Di-O-methoxymethyl-2-C-methyl-D-arabino-γ-lactone (50)

To a solution of 2-methylarabinolactone (**46**) (324 mg, 2 mmol) in CH₂(OMe)₂ (30 mL) and CH₂Cl₂ (30 mL) was added CF₃SO₃H (50 µL), and the solution was stirred at RT under argon for 14 h. The reaction was quenched by addition of 28% NH₄OH (0.1 mL), and the mixture was dried by addition of Na₂SO₄. After removal of the solvent by evaporation, the residue was purified by flash chromatography on silica gel eluting with CH₂Cl₂/MeOH (95:5 to 9:1) to give 450 mg (90%) of product as a pale yellow oil. ¹H-NMR (DMSO-d₆): 6.10 (s, OH, D₂O exchangeable), 4.70 (q, 2H, CH₂), 4.62 (d, 2H, CH₂), 4.30 (m, 1H, H-4), 4.20 (d, 1H, H-3), 3.80-3.65 (m, 2H, H-5), 3.30, 3.28 (2s, 6H, 2 CH₃), 1.26 (s, 3H, CH₃).

### EXAMPLE 12

### 3,5-Di-O-methoxymethyl-2-deoxy-2-fluoro-2-C-methyl-D-ribono-γ-lactone (51)

To a solution of **50** (100 mg, 0.4 mmol) in CH₂Cl₂ (3 mL) and pyridine (0.5 mL) at -78 °C is added DAST or DEOXOFLUOR (0.21 mL, 1.6 mmol), and the solution is stirred at -78 °C for 15 min. Then the solution is allowed to warm up to room temperature and stirred at room temperature for 2 h. The reaction is quenched by addition of saturated aqueous NaHCO₃ (0.5 mL) and ice-water (0.5 mL), followed by CH₂Cl₂ (20 mL) and saturated aqueous NaHCO₃ (10 mL). The aqueous layer is extracted with CH₂Cl₂ twice, the combined organic layers are washed with NaHCO₃, and dried over Na₂SO₄. The evaporation of the solvent gives **51** (88 mg, 87%) as a brownish-yellow oil. ¹H-NMR (DMSO-d₆): 4.74 (q, J = 6.9 & 18.1 Hz, 2H, CH₂), 4.63 (d, J = 0.77 Hz, 2H, CH₂), 4.54 (m, 1H, H-4), 4.18 (dd, J = 7.8 & 20.0 Hz, 1H, H-3), 3.86-3.71 (m, 2H, H-5), 3.34, 3.28 (2s, 6H, 2 CH₃), 1.59 (d, J = 24.26 Hz, 3H, CH₃).

### EXAMPLE 13

### Ethyl 4,5-O-Isopropylidene-3,4,5-trihydroxy-2-methylvalerate (54)

To activated zinc (6.5 g, 0.10 mmol) is added about 20 mL of a solution containing **39** (13.0 g, 0.1 mmol), **53** ( 13.0 mL, 0.10 mmol), THF (50 mL), and diethyl ether (50 mL). After the addition, one crystal of I₂ is added, whereby an exotherm is generated, causing the solution to reflux. The remaining solution is added over about 0.75 h as to maintain a gentle reflux. The mixture is gently heated to reflux for an additional 1 h after the final addition. The mixture is cooled to room temp, poured into ice (200 mL) and 1 N HCl (200 mL) and allowed to stir until most of the ice had melted (about 0.5 h). The organic layer is separated and the aqueous layer is extracted with diethyl ether (2 x 75 mL). The combined organic layers are washed with satd NaHCO₃ (1 x 150 mL), brine (1 x 150 mL), dried (Na₂SO₄), filtered, and concentrated to dryness *in vacuo.* Further drying *in vacuo* provides **54** as a mixture of diastereomers (15.1 g, 65.1%). This compound is used without further purification.

### EXAMPLE 14

### Ethyl 4,5-O-Isopropylidene-3-oxo-2-methylvalerate (55)

Compound **54** (9.85 g, 0.042 mol) is dissolved in dry THF (50 mL). Anhydrous DMSO (16.0 mL, 0.22 mol) is added and the resulting solution is cooled to between -20 °C and -15 °C. Trifluoroacetic anhydride (9.8 mL, 0.69 mol) is added dropwise over 15 minutes and the solution is stirred between -20 °C and -15 °C for 2 h after which anhydrous NEt₃ (24.0 mL, 0.17 mol) is added over 20 min. The resulting solution is stirred at room temp for 1 h, diluted with diethyl ether (50 mL), and washed with H₂O (3 x 100 mL), dried (Na₂SO₄) and concentrate *in vacuo* to compound **55** as a yellow oil (8.1 g, 82.0%) that is used without further purification. ¹H NMR (CDCl₃, 400 MHz): δ 1.24-1.38 (m, 26H), 3.81 (q, 1.3 H, *J* = 7.3 Hz), 3.89 (q, 1.0H, *J* = 7.3 Hz), 3.99-4.04 (m, 3H), 4.10-4.20 (m, 7H), 4.21-4.29 (m, 3H), 4.51 (dd, 1.0H, *J* = 8.1, 6.2 Hz), 4.58 (dd, 1.3H, *J* = 7.7, 5.0 Hz).

### EXAMPLE 15

### Ethyl 4,5 -O-Isopropylidene-2-fluoro-3-keto-2-methylvalerate (56)

Compound **55** (7.36 g, 0.042 mol) is dissolved in anhydrous DMF (5.0 mL) and treated with a slurry of Selectfluor (55.0 g, 0.155 mol) in DMF (45.0 mL). The mixture is placed in an oil bath maintained at 45 - 50 °C and the suspension is maintained with stirring at that temperature overnight under an argon atmosphere. The solution is concentrated to near dryness *in vacuo,* treated with diethyl ether (-25 mL) and washed with water (3 x 100 mL). The organic phase is dried (Na₂SO₄) and *concentrate in vacuo* to compound **56** as a yellow oil (5.65 g, 71.2%) that was an approximate 1:1 mixture of 2R : 2S fluorinated compound as judged by ¹⁹F NMR. ¹H NMR (CDCl₃, 400 MHz): δ 1.20-1.46 (m, 16H), 1.70 (2d, 3H, *J* = 22.8 Hz ), 4.05-4.10 (m, 2H,), 4.12-4.32 (m, 2H,), 4.90-97 (m, 1H). ¹⁹F NMR (CDCl₃, 376 MHz, C₆F₆ external standard): δ 4.30 (q), 4.01 (q).

### EXAMPLE 16

### 3,5-O-dipivaloyl-2-methyl-D-arabino-γ-lactone (47B).

To a solution of **42** (4 mmol, 897 mg) in EtOH (20 mL) was added concentrated HCl (2.0 mL), and the solution stirred at room temperature for 1 h. The solution was concentrated to dryness and the residue was co-evaporated with THF (10 mL) and dissolved in pyridine (6 mL) and CH₂Cl₂ (14 mL). The solution was cooled in ice-bath. To the solution was added pivaloyl chloride (8 mmol, 0.98 mL) and the solution stirred at 0 °C for 30 min. To the solution was added an additional pivaloyl chloride (4 mmol, 0.49 mL) and the solution stirred at room temperature for 5 h. To the solution was added 4-dimethylaminopyridine (100 mg) and the solution was stirred at room temperature for 20 h. H₂O (5 mL) was added and the mixture was stirred at room temperature for 20 min. EtOAc (50 mL) was added. The mixture was washed with water, brine and dried (Na₂SO₄). Solvent was removed and the residue was recrystallized from EtOAc-Hexanes to give fine crystals (625 mg, 47%). H-NMR (CDCl₃): δ 5.18 (d, J = 6.80Hz, 1H, H-3), 4.45, 4.22 (m, 2H, H-5), 4.41 (m, 1H, H-4), 3.32 (br s, 1H, OH, D20 exchangeable), 1.43 (s, 1H, Me), 1.25, 1.22 [ss, 18H, C(Me)₃].

### EXAMPLE 17

### 2-Deoxy-3,5-O-dipivaloyl- 2-fluoro-2-C-methyl-D-ribono-γ-lactone (49B).

To a solution of **47B** (100 mg, 0.3 mmol) in THF (5 mL) were added EtNPr₂ (2 mmol, 0.35 mL) and Deoxo-Fluor (0.18 mL, 0.9 mmol), and the solution was stirred at room temperature for 4 h. To the solution was added additional Deoxo-Fluor (0.18 mL, 0.9 mmol) and the solution was stirred at room temperature for 16 h, refluxed for 1 h. EtOAc (50 mL) was added. The solution was washed with aqueous NaHCO₃, brine, dried (Na₂SO₄). Solvent was removed and the residue was purified by column (10% EtOAc in hexanes) to give product as a solid (65 mg, 65%). H-NMR (CDC1₃): δ 5.12 (m, 1H, H-3), 4.68 (m, 1H, H-4), 4.41, 4.18 (mm, 2H, H-5), 1.63 (d, J = 23.2Hz, 1H, Me), 1.25, 1.20 [ss, 18H, C(Me)₃].

The invention relates to the following items:
1. A 3,5-di-O-protected-2-deoxy-2-fluoro-2-C-methyl-D-ribono-γ-lactone of the following general formula: 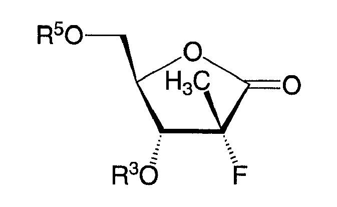 wherein R³ and R⁵ can be independently H, CH₃, or acyl;
   alternatively, R^{3'} and R^{5'} are linked through -SiR₂-O-SiR₂- or -SiR₂-, wherein R is a lower alkyl such as CH₃, ethyl, and n-Pr or I-Pr.
2. A compound of item 1, wherein R³ and R⁵ are each independently H, CH₃, acetyl, benzoyl, pivaloyl, 4-nitrobenzoyl, 3-nitrobenzoyl, 2-nitrobenzoyl, 4-chlorobenzoyl, 3-chlorobenzoyl, 2-chlorobenzoyl, 4-methylbenzoyl, 3-methylbenzoyl, 2-methylbenzoyl, 4-phenylbenzoyl, benzyl, 4-methoxybenzyl, trityl, trialkylsilyl, *t-*butyl-dialkylsilyl, *t-*butyldiphenylsilyl, TIPDS, THP, MOM, or MEM.
3. A process for the preparation of the lactone of Item 1, comprising the steps of:
   (a) reacting a compound of the formula, 39, 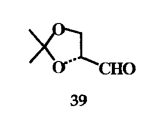 with an alkyl-2-bromopropionate in the presence of activated zinc in a suitable solvent;
   (b) adding an oxidizing agent to provide a ketone;
   (c) fluorinating the product of step (b) to produce a fluorinated ketone;
   (d) reducing the fluorinated ketone of step (c);
   (e) benzylating the product of step (d) to yield the desired lactone.
4. The process of Item 3, wherein the solvent of step (a) is selected from the group consisting of diethyl ether and tetrahydrofuran.
5. The process of Item 3, wherein the oxidizing agent of step (b) is selected from the group consisting of: an activated dimethylsulfoxide, a chromate agent, a Dess-Martin periodione, and tetrapropylammonium perruthenate.
6. A process for the preparation of a lactone intermediate, 46, 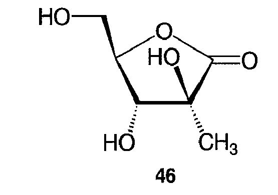 comprising the step of:
   treatment of the compound 42 with concentrated mineral acid 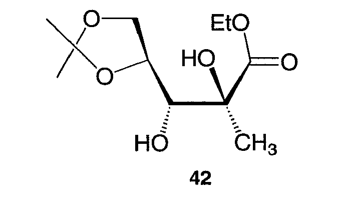
7. A process of preparing a 2'-deoxy-2'-fluoro-2'-C-methyl-β-D-ribofuranosyl nucleoside of the following formula: 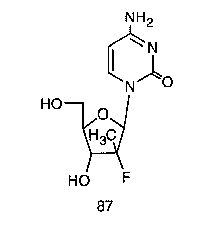 comprising the following steps:
   (a) reducing the lactone of formula 49, 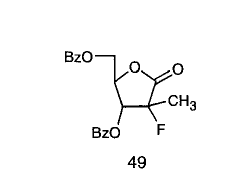 to the corresponding sugar with LiAl(*t-*OBU)₃H, Red-Al, or other aluminum hydride reagents followed by acetylating with an acid anhydride/pyridine or acyl halide to
      yield the compound of formula 59; 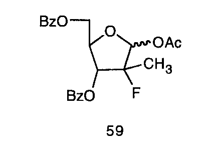
   (b) condensation of the product of step (a), 59, with a silylated
      base to produce a mixture of protected nucleosides, 60 and 60-α; 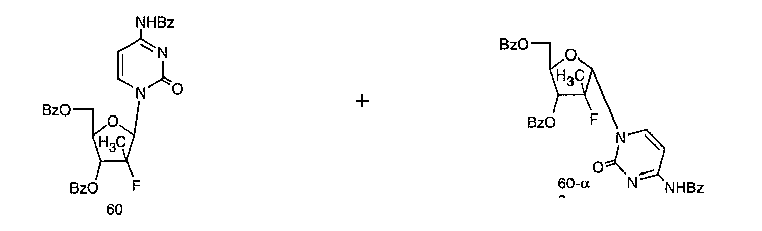
   (c) separation of the anomers of step (b), 60 and 60-α; and
   (d) deprotection of the nucleoside of step (c), 86, to produce the desired nucleoside.
8. The process of Item 7, wherein the silylated base of step (b) is silylated *N*⁴-benzoylcytosine.
9. The process of Item 7, wherein the deprotection of the nucleoside of step (b), 86, is deprotected with a metal alcoholate in alcohol.
10. The process of Item 9, wherein the metal alcoholate in alcohol is NaOMe/MeOH or methanolic ammonia.
11. A process for the preparation of a 2'-deoxy-2'-fluoro-2'-C-methyl-β-D-ribofuranosyl nucleoside of formula 63 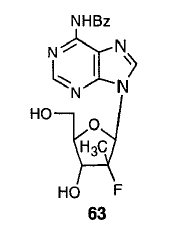 comprising the following steps:
   (a) conversion of a compound of the formula 59, 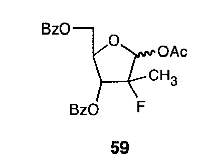 into a bromosugar of formula 61; 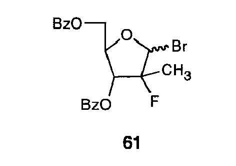
   (b) condensing the product of step (a), 61, with a sodium salt of a
      purine to give the corresponding protected purine nucleoside of formula 62; and 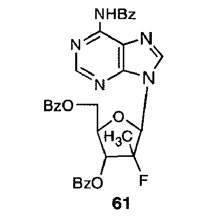
   (c) saponification of the product of step (b), 62, to produce the desired free nucleoside, 63.
12. A process for the preparation of a 2'-deoxy-2'-fluoro-2'-C-methyl-β-D-ribofuranosyl nucleoside of formula 14 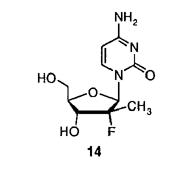 comprising the following steps:
   (a) treating *N⁴*, 5'-O-dibenzoyl-3'-O-mesyl-2'-dioxy-2'-C-methyl-β-D-arabinofuranosylcytocine, 64, 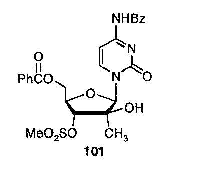 with DAST or Deoxofluor to produce fluorinated product 65, and an olefin, 66; 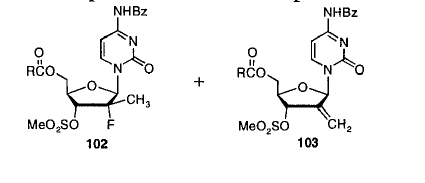
   (b) deprotection of the product of step (a) to yield the desired nucleoside of formula 14.
13. A process for the preparation of a 2'-deoxy-2'-fluoro-2'-C-methyl-β-D-ribofuranosyl nucleoside of formula 14, 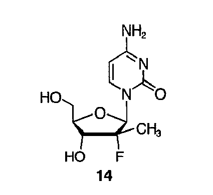 comprising the following steps:
   (a) reacting a nucleoside intermediate of the formula of
      compound 64B, 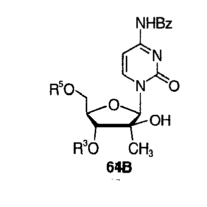 wherein R³ and R⁵ are each independently CH₂OMe, CH₂Ph, MOM, MEM, EOM, p-methoxybenzyl, trityl, acyl of C₁-C₆, aroyl, pivaloyl, alkyl or aryl sulfonate, with DAST or Deoxofluor to yield the fluorinated intermediate, 65B; 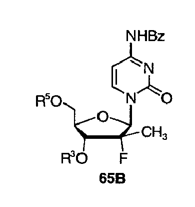
   (b) removing the O-protecting groups of the product of step (a),
      65B, by treatment with a boron trihalide to yield the intermediate, 65C; and 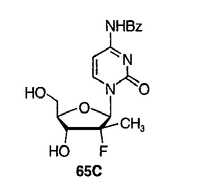
   (c) saponification of the product of step (b), 65C, to yield the desired nucleoside, 14.
14. A process for the preparation of the lactone of Item 1, comprising the steps of:
   (a) reacting a compound of the formula 39 with (1-alkoxycarbonylethylidene)triphenylphosphorane under Wittig conditions in a suitable solvent; 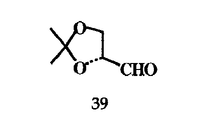
   (b) asymmetric dihydroxylation of the Wittig reaction product 41 to
      provide a diol 42; 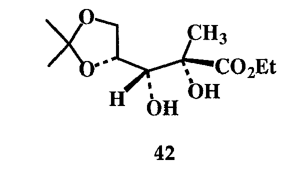
   (c) treatment of the compound 42 with concentrated HCl in ethanol;
   (d) selective O-protection of primary and secondary OH groups
      yielding 3,5- di-O-protected derivative 47; 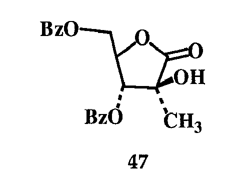
   (e) fluorination of compound 47 provides the desired lactone 49.
15. A process for the preparation of the lactone of Item 1, comprising the steps of:
   (a) selective O-protection of secondary OH group of compound 42
      yield mono-O-protected derivative 43; 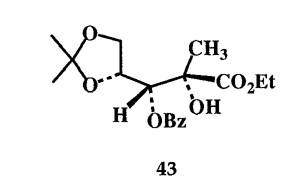
   (b) fluorination of compound 43 provides the fluorinated product 44; 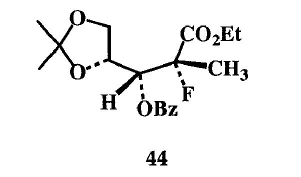
   (c) lactonization of 44 with acid gives the γ lactone 45; 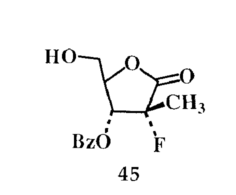
   (d) protection of the primary OH provides the desired lactone 49.
16. A 2-alkyl-4,5-di-O-protected-2,3-dihydroxy-pentanoic-acid ester of the following general formula: 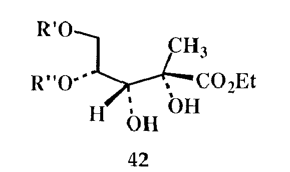 wherein R',R" is isopropylidene ; R' and R" can be independently H, or acyl, benzyl, substituted benzyl, trialkylsilyl, *t-*butyl-dialkylsyl, *t-*butyldiphenylsilyl, TIPDS, THP, MOM, or MEM.
17. An ester according to item 16, wherein acyl is acetyl, benzoyl, pivaloyl, 2,3,4-nitro, chloro or methyl benzoyl, or 4-phenylbenzoyl.
18. A process for the preparation of the diol of the formula, 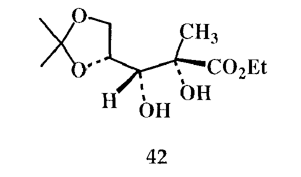 comprising the steps of:
   (a) reacting of a compound of the compound **39** or its analogue 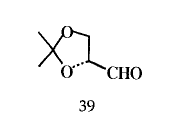 with compound **40** or its analogue 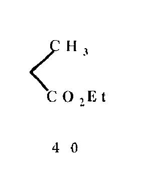 under Wittig conditions in a suitable solvent;
   (b) asymmetric dihydroxylation of the Wittig reaction product **41** 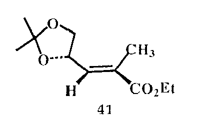 to provide a diol **42.**

## Claims

1. A method for the preparation of a 2'-deoxy-2'-fluoro-2'-C-methyl-β-D-ribofuranosyl nucleoside of the following formula: 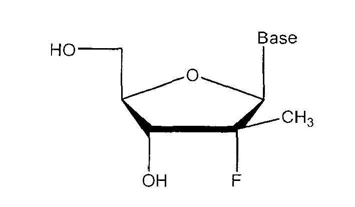 wherein the base is: 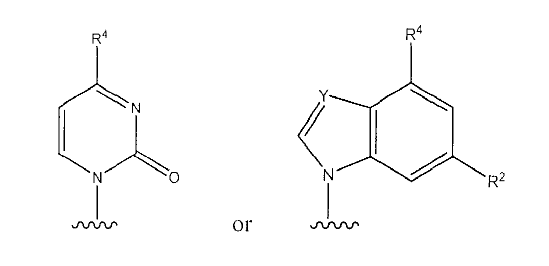 wherein R² and R⁴ are independently H, OH, NH₂, or OR';
R' is an optionally substituted straight, branched, or cyclic alkyl of C₁-C₁₂;
YisNorCH;
comprising the following steps:
(a) reacting a nucleoside intermediate of the following formula: 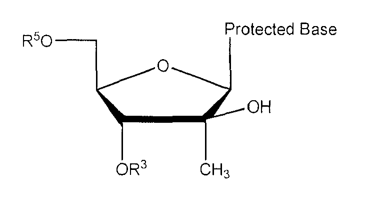 wherein the base is as defined above and the protecting group is benzoyl; wherein R³ and R⁵ are each independently tetrahydropyranyl, methyl, ethyl, benzyl, benzoyl, p-methoxybenzyl, benzyloxymethyl, phenoxymethyl, methoxymethyl, ethoxymethyl, mesyl, tosyl, trifluoroacetyl, or trichloroacetyl with diethylaminosulfur trifluoride or bis(2-methoxyethyl)aminosulfur trifluoride to yield a fluorinated intermediate of the following formula: 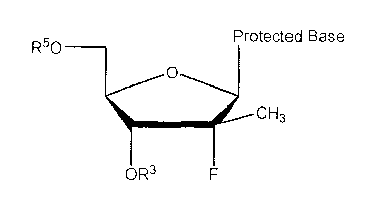
(b) deprotecting the R³ and R⁵ groups of the product of step (a) by treatment with a boron trihalide to yield an intermediate of the following formula: 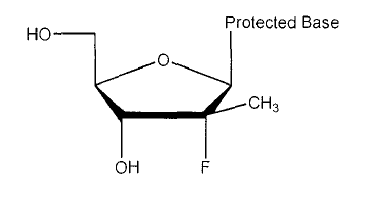
(c) saponifying the product of step (b) to yield the desired nucleoside.

2. The method of claim 1, wherein the boron trihalide of step (b) is selected from boron trifluoride, boron trichloride, boron tribromide or boron triiodide.

3. The method of claim 1, wherein the saponification of step (c) is done with a metal alcoholate in alcohol.

4. The method of claim 3, wherein the metal alcoholate in alcohol is sodium methoxide in methanol.
